# EUROPEAN PATENT APPLICATION

(11) **EP 4 506 072 A1**
(43) Date of publication of application: **12.02.2025**
(21) Application number: 23724000.7
(22) Date of filing: 10.04.2023
(51) Int. Cl.: B07C 5/36, B07C 5/342, G01N 21/85, G01N 21/84

(54) **PORTABLE DEVICE AND METHOD FOR INSPECTING, AND COLLECTING DATA FROM FRUITS AND/OR VEGETABLES IN THE FIELD AND/OR GREENHOUSE**

(30) Priority: 08.04.2022 ES 202230321
(71) Applicant: RULIAT SA, 46550 Albuixech, Valencia (ES)
(72) Inventor: ROQUETA DE LA CARRERA, Bruno, 70000 Uruguay Zona Franca Colonia Sacramento, 70000 (UY)
(74) Representative: Sahuquillo Huerta, Jesús
(86) International application number: PCT/ES2023/070228
(87) International publication number: WO 2023/194643

(57) **Abstract**

A mobile equipment and a method of inspection and grading of product for fruits and vegetables, in field or greenhouse by means of cameras and/or optical sensors and/or other type of product characteristic data collection sensors, comprising: a product inlet (E) and a product outlet (S) at an adjustable height (h), defining between them a zone configured for the passage (1) of a plurality of pieces of produce (F); inspection means (2) comprising at least one camera whose measuring field overlaps with at least a part of the passage zone (1) of a plurality of pieces of produce (F); and wherein the passage zone (1) is inclined an angle (α) such that the product pieces (F) are transferred rotating from the inlet (E) to the outlet (S) by the effect of gravity on a product in an inclined plane.

## Description

### Technical field

The present invention is referred to an inspection equipment for the characterization and classification of fruits and/or vegetables in field and/or greenhouse through artificial vision tools or other data collection sensors.

### State of the art

Before the products (fruits and vegetables) are ready to be displayed on supermarket shelves, they go through a process of harvesting, selection, safety treatments and subsequent packaging, ensuring consumer compliance with sanitary regulations, as well as the homogeneity of the internal and/or external characteristics of the product to be consumed.

The selection process consists of classifying the product according to its intrinsic, external and internal characteristics, such as, but not limited to shape, size, color, specific diameter, weight, cosmetic defects, sugar content and others.

To classify these product characteristics, there are two traditional methods: the first consists of using rollers to move and rotate the product at the same time under the chambers or sensors that collect the information, and it is through this rotation that the entire surface of the product is exposed to the sensors; and the second consists of transporting the product by means of some transport mechanism (belts or other), which drops it in free fall while passing between sensors that take the information from both sides of the product, Both methods have advantages and disadvantages, being used for different applications.

These sorting systems generate large-scale equipment, generally designed to be fixed to the floor and with energy consumption that requires connection to an electrical network or motor generator and are designed to be part of a sorting and packing plant, which makes it impossible or at least unfeasible to transport them to the field for sorting on site during harvesting.

So far, attempts to bring sorting to the field have consisted of reduced versions of these packaging machines that are simplified and placed on a motorized cart that transports them and generates the electricity needed to operate. Although these simplifications reduce the size, weight, and energy consumption, they are still machinery of low mobility and high cost compared to the invention to be presented.

The low mobility and high cost of this equipment in the current state of the art means that the product sampling systems during harvesting are carried out manually, generally with supervisors performing visual inspection of the harvesters, and saving the data on a physical support (paper) or computerized, through hand typing.

There are various documents and examples of machine vision-based product sampling systems and equipment. For example, AU2020102405 describes a method for nondestructive testing of agricultural product quality indicators, which consists of four basic steps: (1) construct a machine vision detection platform, and then collect RGB images of an agricultural product on a vision sensor in all directions; (2) use the image collected in stage (1) to perform image segmentation, and then divide a region of interest for the agricultural product, and extract the contours of the agricultural product; (3) use the agricultural product contours extracted in stage (2), and use a shape invariant feature extraction operator to extract shape feature vectors to overcome the problems of different sizes, positions, and orientations of agricultural products; and (4) use the sample images of the agricultural product with different shapes collected in step (1) and use the feature vectors extracted in steps (2) and (3) to train a support vector machine to determine the optimal parameters and detection accuracy of the support vector machine. However, this paper does not mention how to apply this method in the field itself, during product collection.

On the other hand, being able to measure the contours of the fruit with an image that has not been generated through the union of different images taken during the rotation of the fruit, does not allow the detection of defects, since these may be on the hidden side that has not been taken by the optical sensor.

And in case of generating a complete image of the product without rotating it and with more than one camera, the operation would not be practical and/or feasible to be used during the harvesting process since it would not be a continuous process applicable to large volumes of fruit during harvesting.

Systems that do not generate a total scan of the product are not suitable for defect classification since defects can be located anywhere on the product. Systems that only take a fraction of the product surface can be used for production counting, development evolution, or for classification approximations by variables where extrapolating the characteristics of a smaller area can approximate the characteristics of the entire product, but not for defect classification where it is necessary to be able to see the entire surface.

Another example of using machine vision in agriculture can be found in US 2014/0376771 A1, wherein a system for collecting information about crop growth in a greenhouse is claimed, the system comprising: (a) a first receiving unit configured to receive a first image filming at least one reference crop placed in the greenhouse from a first camera; (b) a second receiving unit configured to receive a second image filming the reference crop from the first camera and a second camera; (c) an information acquisition unit configured to acquire at least one growth data of the reference crop from the first and second image; (d) a database unit storing the acquired growth information; and (e) an estimation unit configured to estimate the growth information of at least one group of crops based on the stored growth information. However, this is not a portable unit for use in field inspection, but a fixed installation in a greenhouse, nor is it clear that scanning of the entire product area can be achieved.

Therefore, the technical problem solved by the present invention is to provide a light, portable, and mobile equipment, which does not require external motorizations for its displacement on site or motor generators for power supply in the field and that can accompany the movements of the work crews during the harvest and a method of product classification in the field by means of artificial vision or other sensors, which allows inspecting, characterizing and classifying in the field the product being harvested and tracing it throughout the production chain to the final consumer.

The absence of this portable device in the current state of the art makes it impossible or at least difficult for producers to have automatic and digital information on the quality of the product being harvested and, consequently:
It is not possible for the Harvest Manager to make real time adjustments in the way of harvesting to avoid hauling unwanted fruit, with the additional costs involved.

It is not possible to give packing plants advance notice of the characteristics of the product being shipped in order to prepare the packing plant for the process prior to the arrival of the fruit, which generates significant improvements in plant efficiency.

It is not possible, or at least difficult, to make specific harvests of product qualities according to the orders placed by customers.

It is not possible to make productivity payments to harvesters in terms of the quality of their work because there is no traceability to measure the quality of the harvester's work.

It is not possible, unless sampling is performed at the packing plant, to know the quality of the product present in the containers that are stored in the cold chambers awaiting processing, and subsequently it is not possible to select containers to be processed whose product quality is aligned with the customers' orders.

It is not possible for independent producers who send their product to be processed in packing plants to corroborate that the information on the quality of the product sent to them and obtained during the inspection and grading process at the packing plant is in line with the quality of the product harvested and shipped.

The way to achieve a correct harvest quality requires the constant supervision of supervisory positions that inspect the work of the crews during harvesting.

These technical problems are solved by the equipment of claim 1.

### Summary of the invention

The present invention has as its object a portable equipment for inspection, characterization, classification and/or sampling of fruits and vegetables with artificial vision or other types of sensors for obtaining data in the field, and which is configured to classify the product according to its external and internal characteristics.

The use of the translation and rotation of a product as a result of the effect of gravity on it when it is positioned on an inclined plane, in order to generate the necessary rotation under the data collection sensors, has not been used so far in the current state of the art, and its use allows the invention of a simple, economical, and portable equipment for massive application in the field during harvesting.

This device can be used for sporadic sampling or directly attached to a container or harvesting vessel so that instead of the harvester dumping his basket or bag of product directly into the container, he does so at the invention's entrance, with all or a large percentage of the harvested product first passing through the invention's inspection before falling into the container or harvesting vessel.

The equipment and method of the invention, in addition, will allow the recording of the data obtained in a local memory or in a remote server, in such a way as to allow the traceability of the product from the very moment when it is collected.

This object is achieved by the apparatus and method of the claims accompanying this specification.

More specifically, mobile and portable equipment for inspection and grading of product selected among fruits and vegetables, in field or greenhouse by means of cameras and/or optical sensors and/or other type of sensors for collecting data characteristic of the product, comprising: a product inlet and a product outlet defining between them a zone configured for the passage of a plurality of pieces of product, the height at the product inlet being always greater than the height at the product outlet;

Means for inspection by means of sensors which may be machine vision or otherwise and which comprise at least one sensor whose field of view overlaps with at least a part of the passage zone of a plurality of product parts; and characterized in that the passage zone is inclined at an angle such that the product parts are rotated from the entrance to the exit by the effect of gravity on a product in an inclined plane.

In a second aspect of the invention, a method of inspecting product in the field using machine vision or other sensors, characterized in that it comprises the steps of:
introducing at least one piece of product freshly picked up in the field into a product inlet of an inspection equipment in the equipment described above, such that said piece of product is transferred rotating by gravity on a product in an inclined device towards a product outlet in a passage area inclined at an angle;
acquiring a data collection sequence of at least one product part moving and rotating under the effect of gravity in the inclined passage zone, wherein the data collection sequence will correspond to a sequence of positions of the product part in the passage zone;
record the data acquired from the product part in a local memory and/or a remote server; and

The product piece classification based on the data extracted and recorded locally or remotely, as well as establishing the traceability of the product piece through the incorporation of a scannable code in the collection container that can then transfer the information to the label of each product during or prior to the process in the packaging, configured to enable remote access to the data of the product piece, generating the possibility for the final consumer to access this information through the scanning of the product label in a supermarket and the subsequent classification of their consumer experience on the product consumed.

Thanks to the use of the following invention it is possible to collect data during harvesting that allows to inspect, characterize and classify the product during harvesting according to its external characteristics such as, but not limited to, equatorial diameter, maximum diameter, average diameter, color, defects, shape, and internal characteristics such as, but not limited to, maturity, bruising and Brix degrees.

By knowing the internal and/or external characteristics of the product present in each container or harvesting container, it is possible, among other things:
make adjustments in real time on the way to harvest avoiding carrying fruit that you do not want to harvest, with the additional costs that this involves.
advance notice to packing plants of the characteristics of the product being shipped in order to prepare the packing plant for the process prior to the arrival of the fruit, which generates significant improvements in plant efficiency.
to carry out specific harvests of product qualities according to the orders placed by customers. making productivity payments to harvesters based on the quality of their work
to know the quality of the product present in the containers that are stored in the cold chambers waiting to be processed, and subsequently to be able to select containers to be processed whose product quality is aligned with the customers' orders.
for independent producers who send their product to be processed in packing plants, corroborate that the information on the quality of the product sent to them and obtained during the inspection and grading process at the packing plant is aligned with the quality of the product harvested and shipped.
eliminating or at least minimizing the supervisory positions that inspect the work of the crews during harvesting operations

The data obtained by the equipment can also be automatically recorded in a local or remote database, which in turn will allow traceability from the harvest to the final consumer who buys the fruit in a supermarket.

Through the use of a label printer attached to the invention, it is possible to print a label identifying the container or container harvester of the product that has been sorted by the invention, and for example, at a stage subsequent to the sorting at the packaging plant, by identifying that container it is possible to know the product being processed and it will be possible to identify - in a non-limiting way - each product with a sticker containing a code scannable by the final consumer (such as, for example, a QR code, a BIDI code, a bar code or any other similar or equivalent ones) that will allow access to the data of said product recorded from the very moment of harvesting. In addition, this allows linking the field data with the packaging data, making its data accessible throughout the product value chain, from the farmer himself, the intermediary, the packer or retailer, and up to the final consumer.

It is important to note that, throughout this description, "field" refers to any space where a product is harvested, and therefore includes not only extensive cultivation plots, but also greenhouses or any other place suitable for growing and harvesting a horticultural and/or vegetable product.

Throughout the description and the claims, the word "comprises" and variants thereof are not intended to exclude other technical features, additives, components or steps. To those skilled in the art, other objects, advantages and features of the invention will be apparent in part from the invention and in part from the practice of the invention. The following examples and drawings are provided by way of illustration and are not intended to restrict the present invention.

Furthermore, the invention covers all possible combinations of particular and preferred embodiments indicated herein.

### Brief description of the drawings

The following is a very brief description of a series of drawings which help to better understand the invention and which relate expressly to an embodiment of said invention, which is illustrated as a non-limiting example thereof.
- FIG.1: shows a schematic of portable equipment for inspection, characterization and classification or sampling of product by means of machine vision or other sensors in accordance with a first aspect of the present invention.
- FIG.2: shows a schematic of a method of sorting and/or sampling product by means of machine vision or other sensors in accordance with a second aspect of the present invention.

### Explanation of a detailed embodiment of the invention

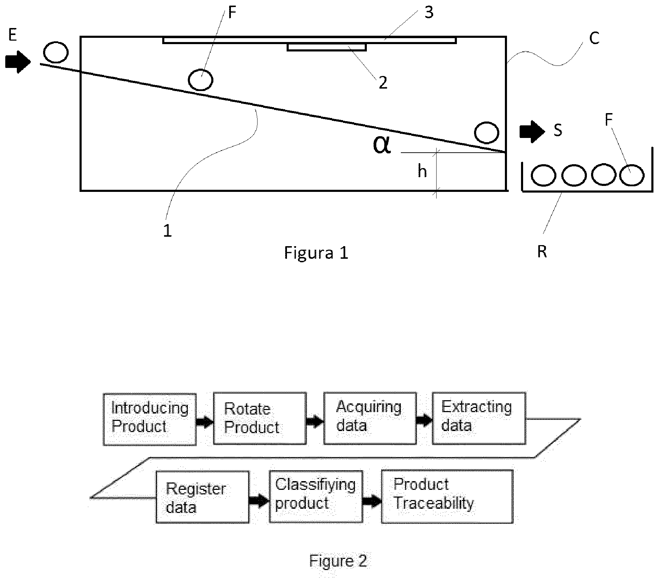

As can be seen in Figure 1, the portable equipment for product sampling in the field by means of artificial vision comprises an inclined device configured for the passage (1) of a plurality of products (F) through a product inlet (E), which allows by the effect of gravity to move and rotate the product to the product outlet (S) where it is transferred to the container (R) without the need of any moving mechanism and which allows to make an analysis of the quality of the product digitally by means of artificial vision means or other sensors (2) comprising at least one sensor and incorporating or not, lighting means (3) and a housing (C).

In addition, the portable sampling equipment has a passage area (1) that is inclined at an angle (α) and adjustable delivery height (h) in such a way that it allows the product pieces (F) to be transferred rotating from the inlet (E) to the outlet (S) by the effect of gravity on the product in an inclined plane, to be then transferred to the container or harvesting container. This feature allows to differentiate from the state of the art in the ability to obtain a view of the entire shell of the product in the field, incorporating a quality that until the present invention could only be found in equipment designed for processing lines and being large, heavy, of very low portability, and also do not have autonomy of operation if they are not connected to an electrical network or to a large engine generator. This differentiation in the way of rotating the product with the absence of rollers or complex mechanical systems allows taking the grading systems to the field, as can be seen in Figure 2, since they do not require large power consumption in addition to the electronics for sensing and data collection, nor do they require motorization to transport the invention or to move and rotate the fruit under the data collection sensors.

In Figure 2, we can observe the steps involved in the portable product sampling equipment in the field, from the moment we introduce a piece of product until we establish the traceability of the piece of product (F). In a first step, we introduce at least one piece of product (F) freshly collected in the field into a product inlet of an inspection equipment; in a second step, said piece of product (F) is transferred and rotated by the effect of gravity in an inclined plane towards an outlet (S) of product in a passage area (1) inclined an angle (α); in a third step, we acquire a sequence of images or readings from other sensors of at least one piece of product (F) translating and rotating under the effect of gravity in the inclined passage zone (1); in a fourth step, we extract and process the data obtained about the product by the sensors and cameras in a local or remote processor, (F) for recording in a local memory and/or a remote server as a fifth step; in a sixth step, we sort the product piece (F) , and then the product is transferred to the receiving device at an adjustable height. Based on the extracted and recorded data, and we establish the traceability of the product part (F). Optionally, by incorporating a scannable code that is printed by an attached printer, it is possible to print a label and stick it on the reception container, which will allow the traceability of the harvested product with its recorded characteristics, and cross-check those results with the subsequent data collected during the sorting process at the packing plant, or share those data with the whole process chain up to the final consumer.

## Claims

1. A mobile and portable product inspection and grading equipment for fruits and vegetables, in the field or greenhouse by means of cameras and/or optical sensors and/or other types of sensors for the collection of product characteristic data, comprising:
a product inlet (E) and a product outlet (S) defining between them a zone configured for the passage (1) of a plurality of pieces of product (F), the height at the product inlet (E) being always greater than the height (h) at the product outlet (S);
inspection means (2) comprising at least one camera or data collecting sensor whose measuring field overlaps with at least a part of the passage area (1) of a plurality of product parts (F); and
**characterized in that** the passage area (1) is inclined at an angle (*α*) so that the product pieces (F) are transferred rotating from the inlet (E) to the outlet (S) by the effect of gravity on a product in an inclined plane.

2. The portable equipment of claim 1 comprising:
One or more processors, at least one memory, and one or more programs, wherein the program or programs are stored in the memory or on some remote server and configured to be executed by the processor or processors, and wherein the programs contain instructions to: **(I)** acquiring a sequence of images or data from other sensors of at least one piece of product (F) at various positions as it is moved and rotated through the inclined passage area (1); **(II)** extracting data from cameras or other sensors of the piece of product (F); and **(III)** recording and/or processing such data in memory or on a remote server.

3. The portable equipment of any one of claims 1 to 2, wherein the angle of inclination (*α*) and/or the output height (h) are fixed or variable between different angles and/or working heights.

4. The portable equipment of any one of claims 1 to 3, wherein the data collection means (2) comprise a plurality of cameras, image sensors or other sensors distributed around the passage area (1) of the product (F).

5. The portable equipment of any one of claims 1 to 3, wherein the data collection means (2) comprises at least one image sensor or other sensor whose position is variable between the product input (E) and the product output (S).

6. The portable equipment of any one of claims 1 to 5 that does not require any type of motorization for transfer in the harvesting field.

7. The portable equipment of any one of claims 1 to 5 to which is added a motorization to be moved in the field.

8. The portable equipment of any one of claims 1 to 7 to which is added a static mechanical device such as curtains in order to control the speed of rotation and/or translation of the fruit on the inclined plane.

9. The portable equipment of any one of claims 1 to 7 to which is added a movable mechanical device in the inclined passage zone (1) as in order to control the speed of rotation and/or translation of the fruit in the inclined plane.

10. The portable equipment of any one of claims 1 to 9 wherein the passageway area (1), in turn, is illuminated by means of illumination means (3).

11. The portable equipment of claim 10 wherein a cabinet or housing (C) is placed on top of the inclined passageway area (1) to block ambient light and to have greater control over the illumination generated by the illumination means (3) on the product.

12. The portable equipment of any one of the preceding claims, comprising a mechanical device at the product outlet (S) configured to separate the outgoing product according to a preselected quality.

13. A method of inspecting product in the field using machine vision or other sensors, **characterized in that** it comprises the steps of:
introducing at least one piece of product (F) freshly collected in the field into a product inlet of an inspection equipment according to any one of claims 1 to 12, such that said piece of product (F) is transferred rotating by the effect of gravity on a product in an inclined device towards an outlet (S) of product in a passage area (1) inclined an angle (*α*);
acquiring a data collection sequence of at least one product part (F) moving and rolling under the effect of gravity in the inclined passage zone (1), wherein the data collection sequence will correspond to a sequence of positions of the product part (F) in the passage zone (1);
record the data acquired from the product part (F) in a local memory and/or a remote server; and
the product piece (F) can be classified according to the data extracted and recorded locally or remotely, as well as establishing the traceability of the product piece (F) by incorporating a scannable code in the collection container (R) that can then transfer the information to the label of each product during or prior to the process in the packaging, configured to enable remote access to the data of the product piece (F), generating the possibility for the final consumer to access this information through the scanning of the product label in a supermarket and the subsequent classification of their consumer experience on the product consumed.
